# EUROPEAN PATENT APPLICATION

(11) **EP 3 184 657 A1**
(43) Date of publication of application: **28.06.2017**
(21) Application number: 15833027.4
(22) Date of filing: 24.08.2015
(51) Int. Cl.: C22B 15/00, C22B 3/18, C12N 1/00, C12R 1/01

(54) **COPPER SULPHIDE LEACHING INFERROUS CHLORIDE MEDIUM WITH BACTERIA**

(30) Priority: 22.08.2014 CL 20142238
(71) Applicant: Compañía Minera Zaldivar Limitada, Santiago (CL)
(72) Inventor: ALVAREZ RODRÍGUEZ, Juan Carlos, Santiago (CL)
(74) Representative: Garavelli, Paolo
(86) International application number: PCT/CL2015/050036
(87) International publication number: WO 2016/026062

(57) **Abstract**

The present invention relates to a method for leaching secondary and primary sulphides of copper in a ferrous chloride medium with iron-oxidising bacteria adapted from the crushing of the mineral from the mine, comprising the steps of: mixing the mineral from the crushing with concentrated sulphuric acid; transporting the material mixed with sulphuric acid by means of a belt; adding a liquid leaching solution at an intermediate point during transport on the belt, said solution consisting of: iron (II) sulphate to reduce the redox potential of the solution-mineral mixture to values less than 550 mV Ag/AgCl; iron (III) sulphate; bacteria and archaea of the mesophile- and thermophile-type belonging to the genera *Acidithiobacillus, Leptospirillum* and *Sulfulobos,* previously adapted in an annexed bioreactor plant; and sodium chloride to produce a chlorinated environment in solutions. The method also comprises the subsequent steps of; heaping the material on head pads to rest for a specific amount of time depending on the mineralogical composition of the material or the operating conditions of the plant; during the resting step, injecting air warmed by a liquid/air exchange system, said air being injected through the lower part of the heap by means of fans; and leaching the heaped material by means of a flow of solutions having a low sulphuric acid content, with ferric ion, which contribute to performing the second step of solubilising the copper sulphides.

## Description

### FIELD OF THE INVENTION

The present invention consists of a process of leaching secondary and primary sulphides of copper in a chloride-ferrous medium with adapted iron-oxidizing bacteria.

### BACKGROUND OF THE INVENTION

The law of copper ore has decreased in many deposits to such an extent that efforts are focused on increasing the mass of processed material in order to maintain copper productions. Facing this challenge, it is necessary to study different techniques in the usual leaching process, which may influence the dissolution of primary and secondary sulphides to increase the kinetics and recovery that these types of ore normally present in comparison to the oxides.

In the case of primary copper sulphides, the common treatment of primary sulphides was prioritized by the mineral concentration line or flotation, especially in copper deposits with laws close to 1% CuT, which makes flotation an attractive process. For primary low-grade reservoir ore (in the order of 0.35% CuT), viability of a concentration process is more difficult, because of the size of the investment capital.

There are several studies that have researched the behavior of the leaching of primary sulphides, which because of their refractory nature are not easily leachable and is a very slow process with recoveries that do not reach 20% in years of irrigation.

In the prior art there are a series of documents that try to solve this problem, in order to improve the recovery of copper from primary sulphides.

Document US 2009/0173188 by Elmar L. Muller, Petrus Basson, Michael J. Nicol, describes a heap leach method for recovering copper from a primary copper sulphide ore in where the mineral is leached in a Chloride/acid-sulfate solution in the presence of oxygen, with the potential of the ore surface below 600 mV to cause the dissolution of copper sulfide.

On the other hand, international application WO 2002/070758 by Budden Julia Rose, Lastra Manuel R, describes an improved process for the bioleaching of copper ores. In this process, the bioleaching takes place below or around Eh <550 mV. Preferably, the concentrations of Fe 2+ and Fe 3+, which are generated during leaching, are maintained so that the concentration of Fe 2+ is greater than Fe 3+. The process has particular utility in relation to chalcopyrite minerals.

The copper recovery method described in WO 2012/001501 by Michael James Nicol, George Frederick Rautenbach, Buuren Craig Van, is related to a copper sulphide ore which is subjected to a leaching chloride cycle, and afterwards to a bioleaching cycle in where the mineral is heated with a chloride-containing chloride-leaching solution.

Document CN103396964 by Zhou Hongbo; Chen Xinhua; Wang Yuguang; Zeng Weimin; Qiu Guanzhou, discloses a composite bacterial community capable of efficiently leaching a sulfur mineral. It further mentions a method of composition and a method of application thereof, and belongs to the metallurgy technique of wet processes. In order to provide a mechanism of biological leaching of the sulfide mineral and according to physiological-biochemical characteristics of the microorganisms, the document mentions a community capable of efficiently leaching sulphide ore, in which the mineral leaching microorganisms comprise Marine bacteria that come from the depths of the sea and are able to withstand high concentration sodium chlorides, oxidized sulfur bacteria, oxidized iron bacteria and archaea that come from a freshwater environment, autotrophic bacteria and facultative heterotrophic bacteria. This solution solves the difficult problem of the intolerance to sodium chloride by the microorganisms leaching minerals from a freshwater environment. The composite bacterial community can increase the leaching efficiency and leaching rate of the sulfide ore, such as copper pyrites.

The above state of the art allows to conclude that the processes described are highly complex and do not achieve high recovery rates.

### SUMMARY OF THE INVENTION

The present invention is based on a process that comprises the inclusion of salts, with the presence of ions such as ferric, ferrous and chloride and all evaluated together with the rest time and temperature and the experiences with bacteria adapted to high levels of chloride.

The addition of ferrous sulfate in specific amounts, immediately after the sulfuric acid curing step, reduces the redox potential of the solution-mineral mixture to values lower than 550 mV Ag/AgCl, avoiding the formation of a passivating layer of the sulphides, especially the primary copper sulphides, where the formation of said layer interferes with subsequent leaching by preventing the direct attack of the oxidizing reagents or leaching agents.

At a redox potential under 550 mV Ag/AgCl, the sulphide ore particles are kept in a permeable form which allows them to be leached by biological and chemical leaching or oxidizing agents. For this reason, the bacteria adapted to chloride that have the ability to indirectly oxidize the ferrous ion to ferric ion are added, allowing the oxidation of the sulfur contained in the mineral matrix. Chemical reagents such as ferric ion and chloride ion, because of their oxidative capacity, solubilize copper sulfide to a compound soluble in acidic solutions.

The proposed process basically consists in lowering the redox potential of the ore bed with ferrous sulfate, adding biological and chemical agents to oxidize or decompose the secondary and primary copper sulphides in the curing step for a subsequent leaching process.

### DESCRIPTION OF THE FIGURES

Figure 1 corresponds to a process carried out by the preferred embodiment of the present invention.
Figure 2 corresponds to a graph showing the recovery of Cu by leaching secondary sulphides with bacteria adapted to high chloride + FeSO₄ levels, according to the preferred embodiment of the invention.
Figure 3 corresponds to a graph showing the recovery of Cu by primary sulfide leaching with bacteria adapted to high concentrations of Chloride + ferrous sulfate, with 3 months of rest, according to the preferred modality of the invention.

### DETAILED DESCRIPTION OF THE INVENTION

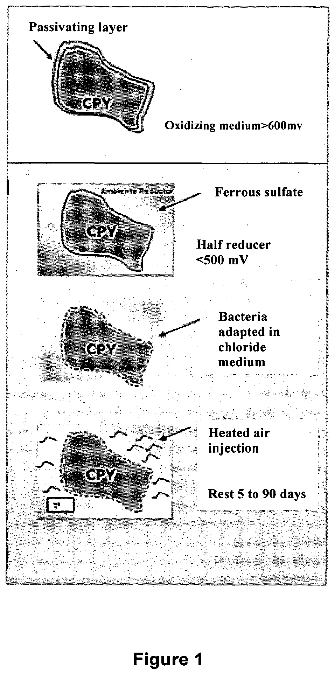

The present invention consists in a process for leaching secondary and primary sulphide copper minerals as shown in Figure 1. The mineralogy on the basis of secondary copper sulphides is predominated by Calcosine and Covelline and in the case of primary sulphides is predominated by Chalcopyrite and Bornite. This process allows to increase the recovery of copper in secondary sulphides and to increase the recovery of copper in primary sulphides predominated in mineralogy by Chalcopyrite.

The process of this invention is applied to minerals with laws between 0.05% to 1.5% for secondary sulphides and 0.05 to 0.5% in case of primary sulphides.

As the first step of the process, the ore is crushed from the mine, which is subjected to a primary, secondary and tertiary crushing operation to reach a desired granulometry, in particular in the order of p80 from 9 to 13 mm.

Subsequently, the material from the tertiary crusher is cured or mixed in a belt with sulfuric acid at a rate between 5 kg/ton to 20 kg/ton of concentrated sulfuric acid. The material cured with sulfuric acid is transported on a belt, during the course of the belt, specifically 10 to 20 meters from the sulfuric acid addition point, where a liquid solution is added preferably composed of the following characteristics:
Lixiviant solution:
   - Ferrous Sulfate: Concentration between 10 gr/L and 100 gr/L
   - Ferric Sulfate: Concentration from 10 to 100 gr/L
   - Bacteria and archaea of the mesophyll and thermophilic type belonging to the genus acidithiobacillus, Leptospirillum and sulfolobos up to a concentration of 10E10 cells/ml. The peculiarity of these microorganisms is that they are adapted in an annexed plant of bioreactors at a high concentration of chloride up to 200 gr/L of chloride.
   - Solution in medium Chloride up to 200 gr/L

The mineral previously cured by acid and lixiviant solution (ferrous, ferric and bacteria adapted to Chloride) continues its course to stacking fields for its later resting step that has a duration between 5 to 90 days, depending on the mineralogical composition of the mineral or Operating conditions of the plant.

In the resting step, air is injected by blowers at the bottom of the stack at a ratio of 0.01 to 0.02 m³ air per ton of mineral. The particularity of this air is that it is heated by at least one liquid / air type heat exchanger with a water circulation process using a heater, preferably electric, as a first step, and heating by means of solar energy in a second step. These steps allow to increase the temperature of the injected air, providing an increase of the internal heat in the stacked material or bed, thus improving the extraction of copper by generating a more favorable environment for the oxidation of the bacterial activity and increasing the kinetics of the Oxidant chemical reaction of the ferric and chloride ions on the sulfated copper ore.

In the preferred embodiment of the present invention, the addition of ferrous sulfate in amounts described above, immediately after the sulfuric acid curing step, reduces the redox potential of the solution-mineral mixture to values less than 550 mV Ag/AgCl, Avoiding the formation of a passivating layer of the sulphides, especially the primary sulphides of copper, since the formation of the layer interferes with the subsequent leaching because it prevents the direct attack of the oxidizing reagents or leaching agents.

A redox potential under 550 mV Ag/AgCl maintains the sulphide ore particles in a permeable form that allows it to be leached by biological and chemical leaching or oxidizing agents. As described above, chloride-adapted bacteria are added, which have the ability to indirectly oxidize the ferrous ion to ferric ion, allowing the oxidation of the sulfur contained in the mineral matrix. Chemical reagents such as ferric ion and chloride ion, because of their oxidative capacity, solubilize copper sulfide to a compound soluble in acidic solutions.

Thus, the present invention basically consists in lowering the redox potential of the ore bed with ferrous sulfate by adding biological and chemical agents to oxidize or decompose the secondary and primary copper sulphides in the curing step for a subsequent leaching process.

Subsequent to the steps described, proceed with the leaching step. The leaching is carried out by entering into the stacked material by means of a flow of low sulfuric acid solutions (4-10 g / It), with ferric ion (1 - 4 g / It), which contributes to the second solubilization step of the copper sulphides.

Leaching provides concentrated Copper solutions, which are then transported to the SX-EW process.

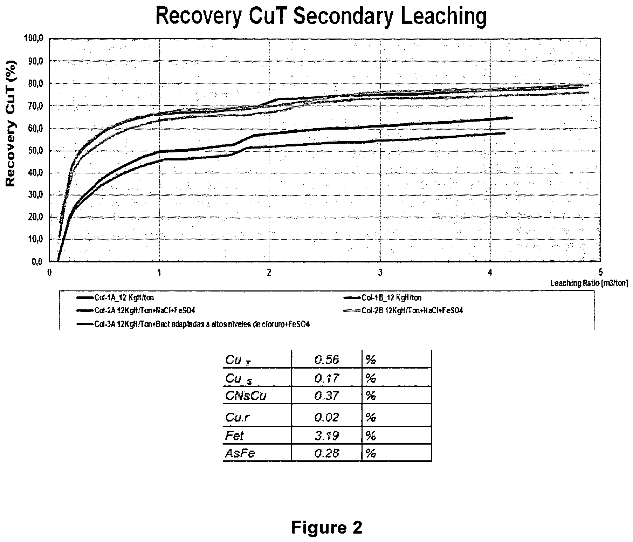

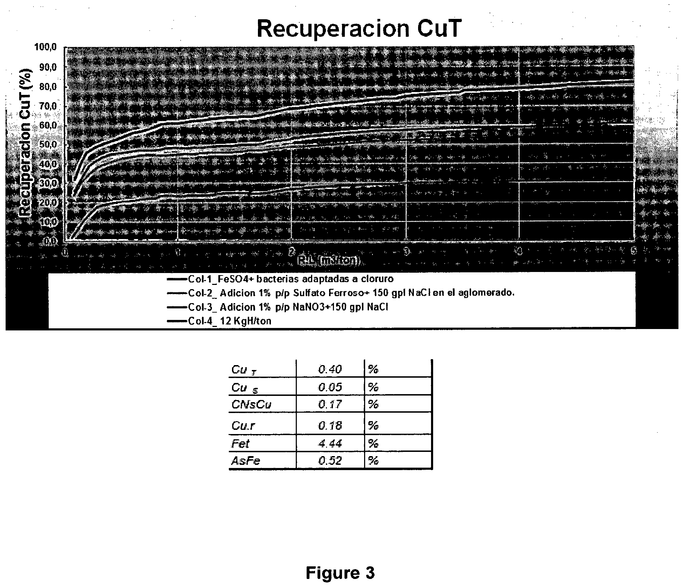

Figures 2 and 3 show copper recovery results by the process described in the present invention with other types of copper recovery processes from secondary primary sulphides. Both figures show the clear increase in copper yield and recovery with the described process.

## Claims

1. A process for leaching secondary and primary sulfides of copper in chloride-ferrous medium with iron-oxidixing bacteria adapted from the crushing of the mineral from the mine, COMPRISING the steps of:
- Mixing the mineral from the crushing with concentrated sulfuric acid at a rate of 5 kg / ton to 20 kg / ton of concentrated sulfuric acid;
- Transporting the material mixed with sulfuric acid in a belt;
- Adding a liquid leach solution at an intermediate point of conveyance of the belt, where said solution is composed of :
∘ Ferrous Sulfate, to reduce the redox potential of the solution-mineral mixture at values lower than 550 mV Ag/AgCI;
∘ Ferric Sulfate;
∘ Bacteria and archaea of the Mesophyll and Thermophilic type belonging to the genus acidithiobacillus, Leptospirillum and sulfolobos, previously adapted in an annexed plant of bioreactors;
∘ Sodium Chloride to generate the chlorinated environment in solutions.
- Stacking the material in stacking fields for a resting step for a determined time duration according to the mineralogical composition of the mineral or operating conditions of the plant;
- Injecting heated air by liquid / air exchange system in the resting step through blowers at the bottom of the stack;
- Leaching the stacked material through a flow of low sulfuric acid solutions with ferric ion, which contribute to the second stage of solubilization of the copper sulphides.

2. The leaching process according to claim 1 wherein the ferrous sulfate concentration ranges from 10 to 100 gr/L.

3. The leaching process according to claim 1 wherein the ferric sulfate concentration ranges from 10 to 100 gr/lt.

4. The leaching process according to claim 1 wherein the concentration of bacteria is up to 10E10 cells/ml.

5. The leaching process according to claim 4 wherein the adaptation of the bacteria is carried out at a high concentration of chloride, which varies up to 200 gr/L,

6. The leaching process according to claim 1 wherein the chloride concentration is up to 200 g/L.

7. The leaching process according to claim 1 wherein the resting step has a duration of from 5 to 90 days

8. The leaching process according to claim 1 wherein air is injected into the stack at a ratio of 0.01 to 0.02 m³ of air per ton of ore.

9. The leaching process according to claim 8 wherein the air is heated by at least one liquid/air type heat exchanger.

10. The leaching process according to claim 1 wherein in the leaching step the sulfuric acid concentration is 4-10 gr/L, with a ferric ion concentration of 1-4 gr/L.
